(19) Europäisches Patentamt
European Patent Office
Office européen des brevets

(11) Veröffentlichungsnummer: **0 309 913 B1**

# (12) EUROPÄISCHE PATENTSCHRIFT

(45) Veröffentlichungstag der Patentschrift: **15.06.94**

(51) Int. Cl.5: **C07D 207/06**, C07D 207/08, C07D 405/04, C07F 7/08, A01N 43/36

(21) Anmeldenummer: **88115569.1**

(22) Anmeldetag: **22.09.88**

Die Akte enthält technische Angaben, die nach dem Eingang der Anmeldung eingereicht wurden und die nicht in dieser Patentschrift enthalten sind.

## (54) Fungizide N-substituierte 3-Aryl-pyrrolidin-Derivate.

(30) Priorität: **30.09.87 DE 3732930**

(43) Veröffentlichungstag der Anmeldung:
**05.04.89 Patentblatt 89/14**

(45) Bekanntmachung des Hinweises auf die Patenterteilung:
**15.06.94 Patentblatt 94/24**

(84) Benannte Vertragsstaaten:
**AT BE CH DE ES FR GB GR IT LI NL SE**

(56) Entgegenhaltungen:
**EP-A- 0 000 333**
**EP-A- 0 007 479**
**EP-A- 0 074 005**
**EP-A- 0 182 224**
**EP-A- 0 244 739**

(73) Patentinhaber: **BASF Aktiengesellschaft**
**Carl-Bosch-Strasse 38**
**D-67063 Ludwigshafen(DE)**

(72) Erfinder: **Zipplies, Matthias, Dr.**
**Kastanienweg 1**
**D-6945 Hirschberg(DE)**
Erfinder: **Sauter, Hubert, Dr.**
**Neckarpromenade 20**
**D-6800 Mannheim 1(DE)**
Erfinder: **Roehl, Franz, Dr.**
**Karl-Otto-Braun-Strasse 8**
**D-6700 Ludwigshafen(DE)**
Erfinder: **Himmele, Walter, Dr.**
**Eichenweg 14**
**D-6909 Walldorf(DE)**
Erfinder: **Ammermann, Eberhard, Dr.**
**Sachsenstrasse 3**
**D-6700 Ludwigshafen(DE)**
Erfinder: **Pommer, Ernst-Heinrich, Dr.**
**Berliner Platz 7**
**D-6703 Limburgerhof(DE)**

EP 0 309 913 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N-substituierte 3-Aryl-pyrrolidine. Verfahren zu deren Herstellung, deren Verwendung als Fungizide, fungizide Mittel, die die neuen Wirkstoffe enthalten sowie Verfahren zur Bekämpfung von Schadpilzen mit diesen Wirkstoffen.

Aus DE 27 27 482 ist das folgende Arylalkylpyrrolidinderivat als Verbindung mit fungizider Wirkung bekannt. Seine Wirkung ist jedoch unbefriedigend.

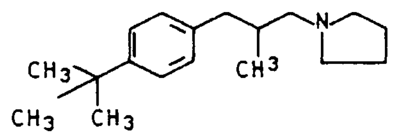

Es ist ferner bekannt, N-substituierte Pyrrolidin- und Piperidinderivate (EP-A-244 739) oder N-Arylpropyl-substituierte cyclische Amine (EP-A-7 479) oder Phenylpropylammoniumsalze von cyclischen Aminen (EP-A-74 005) als Fungizide zu verwenden. Ihre Wirkung ist jedoch unbefriedigend.

Es wurde nun gefunden, daß Verbindungen der Formel I

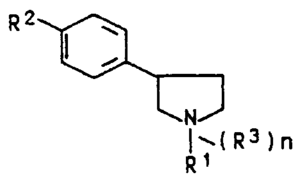

(I) $(X^-)_n$

in der die Substituenten die folgenden Bedeutungen haben

$R^1$ bedeutet 2,2-Dimethyl-propyl, 3,3-Dimethyl-butyl, 4,4-Dimethyl-pentyl. 2,4,4-Trimethyl-pentyl, 6-Methyl-hept-2-yl, 3,5,5-Trimethyl-hexyl, 6,10-Dimethyl-undec-2-yl, 3-Methyl-cyclohexyl, 3,3-Dimethyl-cyclohexyl, 3,3,5-Trimethyl-cyclohexyl, 3,3,5,5-Tetramethyl-cyclohexyl, 4-Methyl-cyclohexyl, 4-Ethyl-cyclohexyl, 4-Propylcyclohexyl, 4-Isopropylcyclohexyl. 4-tert.-Butyl-cyclohexyl, trans-4-tert.-Butyl-cyclohexyl, 4(2-Methyl-but-2-yl)cyclohexyl, 4(2,4,4-Trimethyl-pent-2-yl)-cyclohexyl, Cyclododecanyl, durch C3-$C_9$-Trialkylsilyl-substituiertes $C_4$-$C_{12}$-Cycloalkyl, 4-Hydroxy-cyclohexyl, 4-Hydroxy-3-methyl-cyclohexyl, 4-Hydroxy-3,5-dimethyl-cyclohexyl, 4-Hydroxy-3,3-dimethyl-cyclohexyl,4-Hydroxy-3,3,5-trimethyl-cyclohexyl, gegebenenfalls substituiertes $C_5$-$C_{12}$-Cycloalkenyl mit den Substituenten Hydroxy, $C_1$-$C_9$-Alkyl, $C_1$-$C_5$-Alkoxy, $C_3$-$C_9$-Trialkylsilyl,

$R^1$ bedeutet ferner $C_9$-$C_{11}$-Bicycloalkyl, welches gegebenenfalls substituiert ist durch Hydroxy, $C_1$-$C_6$-Alkyl, $C_1$-$C_5$-Alkoxy, $C_3$-$C_9$-Trialkylsilyl, Acetoxy, Benzoyloxy, Benzyloxy,

$R^1$ bedeutet ferner 4-tert.-Butyl-benzyl, 4-Chlor-benzyl, 4-tert.-Butoxy-benzyl, 1,4-Dioxa-spiro[4,5]decan-8-yl, 5 bis 7-gliedriges Heterocycloalkyl mit 1 oder 2 Heteroatomen aus der Gruppe Sauerstoff und/oder Schwefel,

5 bis 7-gliedriges Heterocycloalkylmethyl mit 1 oder 2 Heteroatomen aus der Grupe Sauerstoff und/oder Schwefel, $C_1$-$C_8$-Alkyl-substituiertes 5 bis 7-gliedriges Heterocycloalkyl oder Heterocycloalkylmethyl mit 1 oder 2 Heteroatmen aus der Gruppe Sauerstoff und/oder Schwefel,

$R^2$ bedeutet $C_3$-$C_{10}$-Alkyl, $C_3$-$C_8$-Alkoxy, $C_3$-$C_9$-Trialkylsilyl,

$R^3$ bedeutet $C_1$-$C_5$-Alkyl, $C_3$-$C_6$-Alkenyl, $C_3$-$C_6$-Alkinyl, $C_7$-$C_{10}$-Arylalkyl, X- bedeutet ein für Pflanzen verträgliches Anion, n den Wert 0 oder 1 hat und deren für Pflanzen verträgliche Salze bei guter Pflanzenverträglichkeit eine hervorragende fungizide Wirkung haben.

Unter Salzen sind Salze mit einem für Pflanzen verträglichen Anion X- mit beliebigen anorganischen und organischen Säuren zu verstehen, z.B. mit Chlorwasserstoff, Fluorwasserstoffsäure, Bromwasserstoff, Schwefelsäure, Phosphorsäure, Jodwasserstoffsäure, Dodecylbenzolsulfonsäure, Ameisensäure, Alkylcarbonsäuren, Essigsäure, Propionsäure, Palmitinsäure, Perfluorheptansäure, Oxalsäure, Malonsäure, Benzoesäure, Äpfelsäure, Dodecylschwefelsäure, Glycerin-2-phosphorsäure, Methylschwefelsäure, Methansulfonsäure, p-Toluolsulfonsäure, Salpetersäure, beispielsweise die folgenden Salze Hydrogensulfat, Dihydrogenphosphat.

Die neuen N-substituierten 3-Aryl-pyrrolidine der Formel I, enthalten chirale Zentren. Sie werden bei ihrer Herstellung im allgemeinen als Racemate oder gegebenenfalls als Diastereomerengemische erhalten.

Reine diastereomere Verbindungen lassen sich bei einigen der neuen Verbindungen beispielsweise durch Destillation, Säulenchromatographie oder aufgrund von Löslichkeitsunterschieden isolieren. Enantiomerenreine Verbindungen lassen sich z. B. durch Racematspaltung mit einem chiralen Hilfsreagenz nach bekannten Methoden beispielsweise über diastereomere Salze erhalten. Für die Anwendung der neuen N-substituierten 3-Aryl-pyrrolidine als Fungizide sind sowohl die Diastereomeren bzw. die Enantiomeren als auch deren bei der Synthese anfallenden Stereoisomerengemische geeignet. Sie alle werden von der Erfindung umfaßt.

$R^1$ bedeutet beispielsweise

2,2-Dimethyl-propyl, 3,3-Dimethyl-butyl, 4,4-Dimethyl-pentyl, 2,4,4-Trimethyl-pentyl, 6-Methyl-hept-2-yl, 3,5,5-Trimethyl-hexyl, 6,10-Dimethyl-undec-2-yl, 3-Methyl-cyclohexyl, 3,3-Dimethyl-cyclohexyl, 3,3,5-Trimethyl-cyclohexyl, 3,3,5,5-Tetramethyl-cyclohexyl, 4-Methyl-cyclohexyl, 4-Ethyl-cyclohexyl, 4-Propyl-cyclohexyl, 4-Isopropylcyclohexyl, trans-4-Isopropyl-cyclohexyl, 4-tert.-Butyl-cyclohexyl, trans-4-tert.-Butyl-cyclohexyl, 4(2-Methyl-but-2-yl)cyclohexyl, 4(2,4,4- trimethyl-pent-2-yl)cyclohexyl, Cyclododecanyl,mit $C_3$-$C_9$-Trialkylsilyl substituiertes $C_4$-$C_{12}$-Cycloalkyl, wie beispielsweise 4-Trimethylsilylcyclohexyl, 4-Hydroxy-cyclohexyl, 4-Hydroxy-3-methyl-cyclohexyl, 4-Hydroxy-3,5-dimethyl-cyclohexyl, 4-Hydroxy-3,3-dimethyl-cyclohexyl, 4-Hydroxy-3,3,5-trimethyl-cyclohexyl,

$C_5$-$C_{12}$-Cycloalkenyl, welches gegebenenfalls durch einen oder mehrere der folgenden Reste substituiert sein kann: Hydroxy, $C_1$-$C_9$-Alkyl, $C_1$-$C_5$-Alkoxy, C-$C_9$-Trialkylsilyl, wie beispielsweise 4-tert.-Butyl-cyclohexenyl, 4-tert.-Butoxy-cyclohexenyl, 4-Trimethylsilyl-cyclohexenyl;

$C_9$-$C_{11}$-Bicycloalkyl, welches gegebenenfalls durch einen oder mehrere der folgenden Reste substituiert sein kann: Hydroxy, $C_1$-$C_6$-Alkyl, $C_1$-$C_5$-Clkoxy, $C_3$-$C_9$-Trialkylsilyl, Acetoxy, Benzyloxy, Benzoyloxy, wie beispielsweise [4.3.0]-Bicyclononyl, Decalyl, 9-Methyl-2-decalyl, 5,9-Dimethyl-2-decalyl, 5,5,9-Trimethyl-2-decalyl, 6-Hydroxy-2-decalyl, 6-Benzoyloxy-2-decalyl, 7-Hydroxy-2-decalyl, 6-Hydroxy-9-methyl-2-decalyl, 6-Hydroxy-5,9-dimethyl-2-decalyl, 6-Hydroxy-5,5,9-trimethyl-2-decalyl, 6-Benzoyloxy-5,9-dimethyl-2-decalyl,

5 bis 7-gliedriges Heterocycloalkyl mit 1 oder 2 Heteroatomen aus der Gruppe Sauerstoff oder Schwefel, wie beispielsweise Tetrahydropyranyl, Tetrahydrothiopyranyl, Dioxanyl,

5 bis 7-gliedriges Heterocycloalkylmethyl mit 1 oder 2 Heteroatomen aus der Gruppe Sauerstoff oder Schwefel, z.B. Tetrahydropyranylmethyl, Dioxanylmethyl,

$C_1$-$C_8$-Alkyl-substituiertes 5 bis 7-gliedriges Heterocycloalkyl oder Heterocycloalkylmethyl mit 1 der 2 Heteroatomen aus der Gruppe Sauerstoff oder Schwefel, wie beispielsweise Tetrahydropyranylmethyl, Dioxanylmethyl, 3,5-Dimethyl-dioxan-2-yl-methyl, Diethyl-dioxan-2-yl-methyl, 2-Isopropyl-1,3-dioxan-5-yl, 2-tert.-Butyl-1,3-dioxan-5-yl, $R^1$ bedeutet ferner 4-tert.-Butylbenzyl, 4-Chlor-benzyl, 4-tert.-Butoxybenzyl, 1,4-Dioxa-spiro[4.5]decan-8-yl.

$R^2$ bedeutet beispielsweise verzweigtes oder unverzweigtes $C_3$-$C_{10}$-Alkyl, wie beispielsweise Propyl, Isopropyl, sek. Butyl, tert. Butyl, 2-Methyl-but-2-yl, 2,4,4-Trimethyl-pent-2-yl, verzweigtes oder unverzweigtes $C_3$-$C_8$-Alkoxy, wie beispielsweise Propyloxy, iso-Propoxy, Butyloxy, tert.-Butyloxy, $C_3$-$C_9$-Trialkylsilyl, wie beispielsweise Trimethylsilyl.

$R^3$ bedeutet beispielsweise $C_1$-$C_5$-Alkyl, wie beispielsweise Methyl, Ethyl, Propyl, Butyl, Pentyl, $C_3$-$C_6$-Alkenyl, wie beispielsweise Allyl, Methallyl, $C_3$-$C_6$-Alkinyl, wie beispielsweise Propargyl, $C_7$-$C_{10}$-Arylalkyl wie beispielsweise Benzyl, Phenylethyl.

Die Verbindungen der Formel I können beispielsweise hergestellt werden durch

- N-Alkylierung von Pyrrolidinen der Formel II
- für den Fall, daß $R^2$ = Alkyl ist, durch Friedel-Crafts-Alkylierung von N-alkylierten Phenyl-pyrrolidinen der Formel VI
- den fünfgliedrigen Heterocyclus aufbauende Reaktionen und anschließende Reduktion.

Die Verfahren werden im folgenden erklärt:

Einführung des Restes $R^1$.

a) Umsetzung eines Pyrrolidinderivates II mit einer Verbindung $R^1$-X unter basischen Bedingungen.

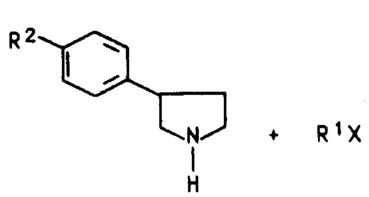

(II)

Als Reste X seien beispielsweise genannt, Chlor, Brom, Jod, die Methan-, Benzol-, p-Toluol-sulfonylgruppe oder die den oben genannten Anionen X-entsprechenden Reste.

Die Reaktion wird beispielsweise bei 40-200 ° C, gegebenenfalls in Gegenwart eines inerten Lösungsmittels durchgeführt. Als Basen werden anorganische Basen bevorzugt, beispielsweise Kalium-, Natrium- und Lithiumhydroxid, Natriumhydrid, Kalium- und Natriumcarbonat. Geeignet sind auch organische Basen wie Triethylamin, Dicyclohexylamin und Diisopropylamin. Die Reaktion läßt sich auch mit einem Überschuß des Pyrrolidinderivates II durchführen.

b) Umsetzung einer Carbonylverbindung III

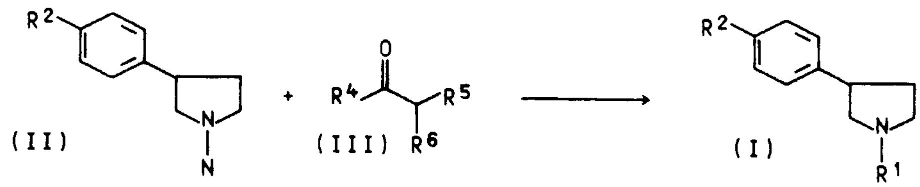
(II) (III) (I)

wobei die Reste $R^4$ bis $R^6$ so definiert sind, daß der Rest,

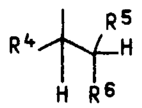

in seiner Gesamtheit dem Rest $R^1$ entspricht, mit einem Pyrrolidinderivat II unter gleichzeitiger oder anschließender Reduktion bzw. Hydrierung.

$b_1$) Bei der direkten Methode zur Herstellung der Verbindung I wird ein Gemisch aus II und III in Gegenwart eines Lösungsmittels, beispielsweise Methanol, Ethanol, Propanol, Isopropanol, die bis zu 25 % (Vol.) Wasser enthalten können, mit Natriumcyanborhydrid oder Natriumborhydrid, gegebenenfalls in Gegenwart eines Metallsalzes wie Zink(II)chlorid, Cadmium(II)chlorid oder Magnesium(II)chlorid, bei 0-100 ° C, vorzugsweise 20-80 ° C oder aber in Gegenwart eines Lösungsmittels wie beispielsweise Methanol, Ethanol, Tetrahydrofuran oder Toluol und eines Hydrierkatalysators wie z. B. Raney-Nickel, Platin-IV-oxid, $Ru_2O_3$ oder Palladium auf Kohle im Autoklaven bei 100-150 ° C mit Wasserstoff bis Druckkonstanz umgesetzt.

$b_2$) Bei der Zweistufen-Reaktion wird aus den Verbindungen II und III ein Enamin in an sich bekannter Weise unter wasserentziehenden Bedingungen hergestellt und anschließend mit einem Edelmetallkatalysator wie Raney-Nickel, Raney-Cobalt, $PtO_2$ oder $Ru_2O_3$, vorzugsweise Palladium auf Kohle und Wasserstoff reduziert.

c) Für den Fall, daß $R^2 = C_3$-$C_{10}$-Alkyl ist, läßt sich $R^2$ in ein Phenylpyrrolidin-Derivat, Struktur VI

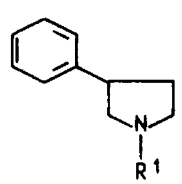
(VI)

einführen, indem man dieses säurekatalysiert mit einem Alken der Struktur Va,

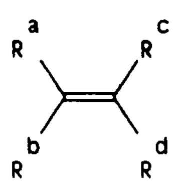
(Va)

in welchem die Reste $R^a$-$R^d$ so definiert sind, daß der Rest Vb in seiner Gesamtheit

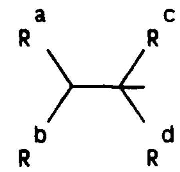

(Vb)

dem Rest $R^2$ entspricht, bzw. mit einem Alkylhalogenid $R^2$-X umsetzt. Man verwendet Mineralsäuren, wie beispielsweise Schwefelsäure, Salzsäure, Bromwasserstoffsäure oder auch Lewis-Säuren wie $AlCl_3$ oder $SnCl_4$ und führt die Reaktion unter Eiskühlung z. B. bei -10 bis +20 °C durch.

d) Ringaufbauende Reaktionen

Die Verbindungen I lassen sich auch durch Umsetzungen von primären Aminen der Formel $R^1$-$NH_2$ mit einer Verbindung VIIIa, VIIIb, VIIIc und anschließender Reduktion der Carbonylgruppe(n) herstellen.

Bei der Umsetzung von Dicarbonsäuren, Bernsteinsäureanhydriden und 5-Ringlactonen wird das Reaktionswasser entweder am Wasserabscheider mit einem geeigneten Schleppmittel oder in Gegenwart eines wasserbindenden Mittels aus dem Gleichgewicht entfernt. Als Lösungsmittel

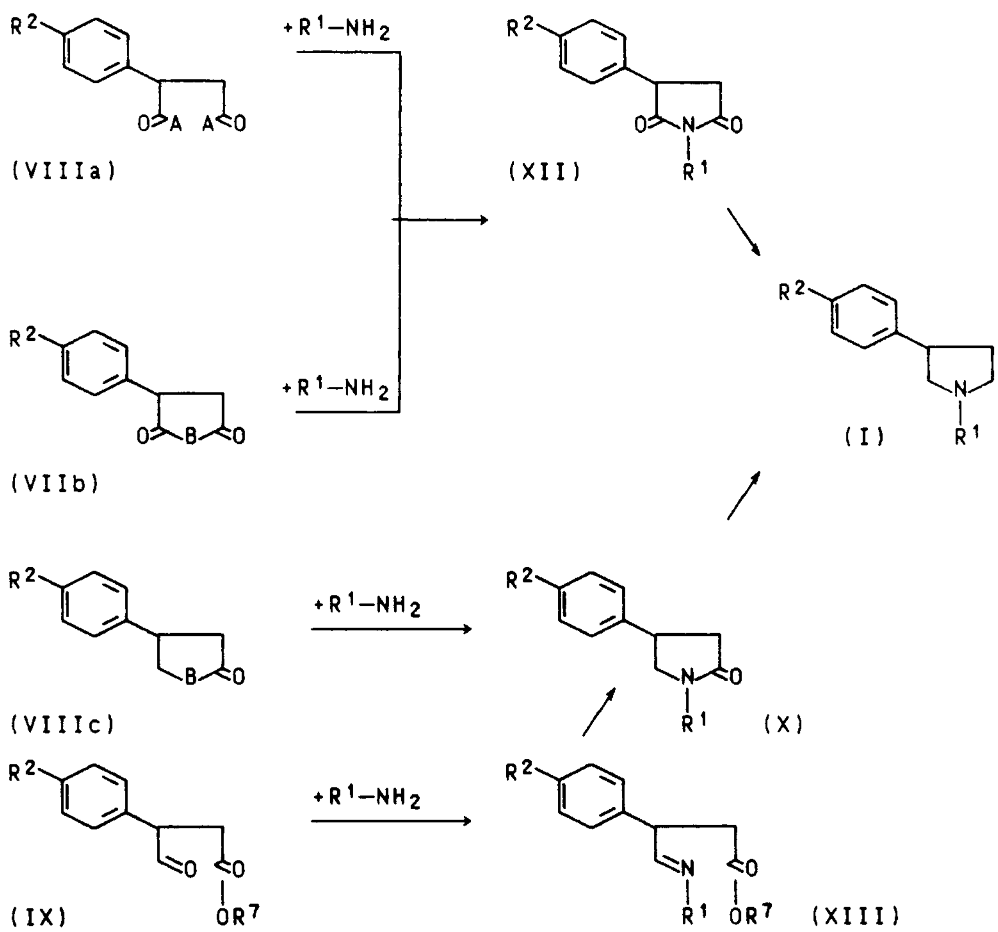


A = Hydroxy, Alkoxy, Halogen

B = O oder NH

$R^7$ = Alkyl

bei der Wasserabscheidung eignen sich höher siedende Kohlenwasserstoffe wie Toluol, Xylol, Chlorbenzol oder Ligroin. Als wasserbindende Mittel eignen sich Aceton oder Molekularsiebe.

Es kann hierbei aber auch ohne Lösungsmittel gearbeitet werden. Die Reaktionen lassen sich bei Temperaturen ab 100 °C durchfuhren. Falls die Reaktionstemperatur unter Normaldruck nicht erreichbar ist, wird im Autoklaven unter dem Eigendruck der Reaktionsmischung bei der benötigten Umsatztemperatur gearbeitet.

Die Dicarbonsäurendihalogenide lassen sich bei -20 bis +100 °C, vorzugsweise bei 0 bis +10 °C in Gegenwart einer Base, die zusätzlich als Lösungsmittel dienen kann, wie beispielsweise Diisopropylamin, Tributylamin, Pyridin, Picolin, Triethylamin, Dicyclohexylamin oder einer Base wie beispielsweise Natriumcarbonat, Kaliumcarbonat oder Natriumhydrogencarbonat in Gegenwart eines inerten Lösungsmittels wie Chloroform, Dichlormethan oder Tetrahydrofuran umsetzen.

Die Umsetzung der Aminderivate $R^1$-$NH_2$ mit einem 5-Ring-Lacton-Derivat (VIIIc, B = NH) findet bei hohen Temperaturen, die zwischen 150 und 280 °C liegen, statt, wobei die benötigte Reaktionstemperatur gegebenenfalls in einem Autoklaven erzielt werden kann. Die Reaktion kann in einem inerten Lösungsmittel wie beispielsweise Toluol, Xylol, Ethanol, Isopropanol oder Cyclohexanol erfolgen oder auch in Abwesenheit eines Lösungsmittels.

Zur Reduktion der Carbonylverbindung zur Alkylverbindung kann man mit einem Reduktionsmittel bei -20 bis +100 °C, vorzugsweise 0 bis +60 °C umsetzen. Als Reduktionsmittel finden bevorzugt Hydride wie Lithiumaluminiumhydrid und Diboran Verwendung.

Die Reduktion kann auch unter Wolff-Kishner-Reaktionsbedingungen oder elektrochemisch erfolgen.

Geeignete Lösungsmittel für die Hydridreaktion sind Ether wie Diethylether, Diisopropylether, Methyl-tert.-Butylether, Tetrahydrofuran und Dioxan, Kohlenwasserstoffe, wie Xylol, Toluol.

Die 3-Formyl-propionsäureester-Derivate der allgemeinen Struktur IX lassen sich bei 0-100 °C, gegebenenfalls in Gegenwart eines wasserentziehenden Mittels wie Natriumsulfat, Magnesiumsulfat oder Molekularsieb, oder aber auch am Wasserabscheider mit einem geeigneten Schleppmittel mit den Aminen $R^1$-$NH_2$ zu den Schiffschen Basen der allgemeinen Struktur XIII umsetzen.

Als Lösungsmittel eignen sich chlorierte Kohlenwasserstoffe, wie Dichlormethan, Ether, wie Tetrahydrofuran und Kohlenwasserstoffe, wie Toluol oder Xylol.

Die Verbindungen XIII lassen sich in an sich bekannter Weise mit Reduktionsmitteln wie Natriumborhydrid oder Lithiumaluminiumhydrid oder aber auch an Katalysatoren wie Palladium-Aluminiumoxyd oder Raney-Nickel mit Wasserstoff in die Pyrrolidon-Derivate X überfuhren. Diese lassen sich, wie oben beschrieben, mit Reduktionsmitteln, wie beispielsweise Lithiumaluminiumhydrid in Ether oder Dioxan, zu den erfindungsgemäßen Verbindungen I umsetzen.

Die Erfindung umfaßt ferner Verfahren zur Herstellung der quartären Salze durch Umsetzung der Pyrrolidinderivate der Formel I mit der Verbindung $R^3$-X.

Als Reste X seien beispielsweise genannt Chlor, Brom, Jod, die Methan- und p-Toluolsulfonylgruppe, Methylsulfat. Die Reaktion wird bei 10-200 °C, gegebenenfalls in Gegenwart eines inerten Lösungsmittels durchgeführt. Als Lösungsmittel eignen sich Kohlenwasserstoffe wie Dekalin, Toluol, Xylol, chlorierte Kohlenwasserstoffe wie Chlorbenzol, Ether, wie Tetrahydrofuran, Dioxan, dipolar aprotische Lösungsmittel wie Dimethylsulfoxid, Dimethylformamid, Sulfolan, Acetonitril, Alkohole wie Methanol, Ethanol, Propanol, Butanol, Amylalkohol; die Reaktion läßt sich jedoch auch ohne Lösungsmittel durchführen. Das bei der Herstellung gebildete Gegenion $X^-$ kann mittels eines Anionenaustauschers oder durch Umfällungsreaktion gegen ein anderes geeignetes Anion ausgetauscht werden.

Z.B. läßt sich 3-Phenylpyrrolidin mit Isobutylen in einem chlorierten Kohlenwasserstoff wie Dichlormethan, Tetrachlorkohlenstoff, Tetrachlorethan, Chlorbenzol oder anderen geeigneten Lösungsmitteln wie Schwefelkohlenstoff oder Nitrobenzol Säurekatalysiert zu I ($R^2$ = tert.-Butyl) umsetzen.

Man verwendet Mineralsäuren wie beispielsweise Schwefelsäure als Katalysatoren und führt die Reaktion unter Eiskühlung bei -5 °C bis +20 °C durch.

Die Ausgangsstoffe sind bekannt oder in an sich bekannter Weise darstellbar.

Die folgenden Beispiele erläutern die Herstellung der neuen Verbindungen:

Herstellungsbeispiele

Beispiel 1

3(4-tert.-Butyl-phenyl)pyrrolidin

Zu 29,4 g (0,2 mol) 3-Phenyl-pyrrolidin in 400 ml Tetrachlorkohlenstoff wurden bei 0-5 °C 98 g (1 mol) konz. Schwefelsäure zugetropft. Nach 15 Min. wurden 15,2 g (0,27 mol) Isobutylen eingegast, 1 Std. bei 0-5 °C gerührt und bei 20 °C mit 400 ml Wasser hydrolysiert. Die organische Phase wurde abgetrennt und die wäßrige Phase mit 2 x 100 ml Dichlormethan extrahiert.

Die Aufarbeitung der organischen Phasen ergab 42 g eines Rohprodukts, das i.Vak. fraktioniert wurde: Sdp. : 84-87 °C/0,5 mbar; 13,4 g (33 %) eines farblosen Öls.

Beispiel 2

N(4-tert.-Butyl-cyclohexyl)-3(4-tert.-butyl-phenyl)pyrrolidin (Verbindung Nr. 16)

15,4 g (100 mmol) 4-tert.-Butyl-cyclohexanon, 9,8 g (48 mmol) 3(4-tert.-Butyl-phenyl)pyrrolidin, 6,8 g (50 mmol) Zink(II)chlorid und 6,3 g (100 mmol) Natriumcyanborhydrid wurden in 200 ml absolutem Methanol 48 Std. bei 22 °C gerührt. Das Methanol wurde i.Vak. abdestilliert und der Rückstand in Dichlormethan und 5 %-iger Natronlauge gelöst.

Die Aufarbeitung des organischen Extrakts und Fraktionierung i.Vak. (175-80 °C/0,4 mbar) lieferte ein Isomerengemisch, welches an Kieselgel mit Hexan/Methyl-tert.-butyl-ether chromatographiert wurde:

N(cis-4-tert.-Butyl-cyclohexyl)-3(4-tert.-butyl-phenyl)pyrrolidin Schmp. 58 °C (Verb. 16b) : 2,3 g (14 %).

N(trans-4-tert.-Butylcyclohexyl)-3(4-tert.-butyl-phenyl)-pyrrolidin, Schmp. 50-55 °C (Verb. Nr. 16a): 2,5 g (15 %).

Die folgenden Verbindungen können in entsprechender Weise hergestellt werden.

Tabelle 1:

n = 0

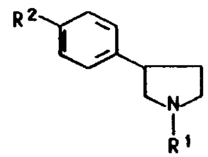

| Verb.-Nr. | $R^1$ | $R^2$ | Sdp.($^0C$/mbar) | IR-Absorption ($cm^{-1}$) [Film] |
|---|---|---|---|---|
| 1 | 2,2-Dimethyl-propyl | tert.-Butyl | Öl | 2954, 2905, 2867, 2780, 1478, 1464, 1394, 1361, 1141, 1121, 828, 572 |
| 2 | 3,3-Dimethyl-butyl | tert.-Butyl | Öl | 2956, 2867, 1474, 1466, 1364, 1145, 828, 573 |
| 3 | 4,4-Dimethyl-pentyl | tert.-Butyl | 140/0,4 | |
| 4 | 2,4,4-Trimethyl-pentyl | tert.-Butyl | Öl | 2956, 2909, 2868, 2787, 1476, 1465, 1393, 1383, 1364, 1268, 828, 573 |
| 5 | 6-Methyl-hept-2-yl | tert.-Butyl | 150-154/0,4 | |
| 6 | 3,5,5-Trimethylhexyl | tert.-Butyl | 152-155/0,4 | |
| 7 | 6,10-Dimethyl-undec-2-yl | tert.-Butyl | Öl | 2956, 2927, 2867, 1464, 1377, 1366, 1169, 820 |
| 8 | 3-Methyl-cyclohexyl | tert.-Butyl | | |
| 9 | 3,3-Dimethyl-cyclohexyl | tert.-Butyl | 160-170/0,4 | |
| 10 | 3,3,5-Trimethylcyclohexyl | tert.-Butyl | | |
| 11 | 3,3,5,5-Tetramethyl-cyclohexyl | tert.-Butyl | | |
| 12 | 4-Methyl-cyclohexyl | tert.-Butyl | | |
| 13 | 4-Ethyl-cyclohexyl | tert.-Butyl | | |
| 14 | 4-Propyl-cyclohexyl | tert.-Butyl | | |
| 15 | 4-Isopropyl-cyclohexyl | tert.-Butyl | Öl | 2958, 2934, 2865, 2777, 1464, 1449, 1369, 1366, 1152, 828 |
| 16 | 4-tert.-Butyl-cyclohexyl | tert.-Butyl | | |

| Verb.-Nr. | $R^1$ | $R^2$ | Sdp.($^0$C/mbar) | IR-Absorption ($cm^{-1}$) [Film] |
|---|---|---|---|---|
| 16a | trans-4-tert.-Butyl-cyclohexyl | tert.-Butyl | Fp 50-55 | |
| 16b | cis-4-tert.-Butyl-cyclohexyl | tert.-Butyl | Fp 58 | |
| 17 | 4(2-Methyl-but-2-yl)-cyclohexyl | tert.-Butyl | | |
| 18 | 4(2,4,4-Trimethyl-pent-2-yl)-cyclohexyl | tert.-Butyl | | |
| 19 | Cyclododecanyl | tert.-Butyl | Harz | 2932, 2863, 2849, 2776, 1472, 1446, 1362, 1121, 828, 572 |
| 20 | 4-Trimethylsilyl-cyclohexyl | tert.-Butyl | Fp 75-79 | |
| 21 | 4-Hydroxy-cyclohexyl | tert.-Butyl | | |
| 22 | 4-Hydroxy-3-methyl-cyclohexyl | tert.-Butyl | | |
| 23 | 4-Hydroxy-3,5-dimethyl-cyclohexyl | tert.-Butyl | | |
| 24 | 4-Hydroxy-3,3-dimethyl-cyclohexyl | tert.-Butyl | | |
| 25 | 4-Hydroxy-3,3,5-trimethyl-cyclohexyl | tert.-Butyl | Öl | 2961, 2868, 2778, 1476, 1460, 1363, 1338, 1058, 1038, 980 |
| 26 | 4-tert.-Butyl-cyclohex-3-en-yl | tert.-Butyl | | |
| 27 | 4-tert.-Butyl-cyclohex-2-en-yl | tert.-Butyl | | |
| 28 | 1-Decalyl (cis/trans-Gemisch) | tert.-Butyl | | |
| 29 | 2-Decalyl (cis/trans-Gemisch) | tert.-Butyl | 158-162/0,4 | |
| 30 | trans-2-Decalyl (eq/ax.-substituiert) | tert.-Butyl | | |
| 31 | eq.-trans-2-decalyl | tert.-Butyl | | |
| 32 | 6-Hydroxy-2-decalyl | tert.-Butyl | | |
| 33 | 7-Hydroxy-2-decalyl | tert.-Butyl | | |
| 34 | 2-Decalylmethyl | tert.-Butyl | | |
| 35 | 9-Methyl-trans-2-decalyl | tert.-Butyl | | |
| 36 | 5,9-Dimethyl-trans-2-decalyl | tert.-Butyl | | |
| 37 | 5,5,9-Trimethyl-trans-2-decalyl | tert.-Butyl | | |
| 38 | 6-Hydroxy-9-methyl-2-decalyl | tert.-Butyl | | |
| 39 | 6-Hydroxy-5,9-dimethyl-2-decalyl | tert.-Butyl | | |
| 40 | 6-Hydroxy-5,5,9-trimethyl-2-decalyl | tert.-Butyl | | |

| Verb.-Nr. | $R^1$ | $R^2$ | Sdp.(°C/mbar) | IR-Absorption ($cm^{-1}$) [Film] |
|---|---|---|---|---|
| 41 | Tetrahydropyran-4-yl | tert.-Butyl | 125-130/0,4 | |
| 42 | Tetrahydropyran-4-yl | tert.-Butyl | | |
| 43 | Dioxan-2-yl-methyl | tert.-Butyl | | |
| 44 | Tetrahydropyran-2-yl-methyl | tert.-Butyl | | |
| 45 | Tetrahydropyran-3-yl-methyl | tert.-Butyl | | |
| 46 | 3,5-Dimethyl-dioxan-2-yl-methyl | tert.-Butyl | | |
| 47 | 3,5-Diethyl-dioxan-2-yl-methyl | tert.-Butyl | | |
| 48 | 3,6-Diethyl-dioxan-2-yl-methyl | tert.-Butyl | | |
| 49 | 2-Isopropyl-1,3-dioxan-5-yl | tert.-Butyl | | |
| 50 | 2-tert.-Butyl-1,3-dioxan-5-yl | tert.-Butyl | | |
| 51 | 4-tert.-Butyl-cyclohexyl | tert.-Butyl | | |
| 52 | trans-4-tert.-Butyl-cyclohexyl | tert.-Butyl | | |
| 53 | 4-tert.-Butoxy-cyclohexyl | tert.-Butyl | | |
| 54 | 4-tert.-Butyl-benzyl | tert.-Butyl | Fp 89-94 | |
| 55 | 4-Chlor-benzyl | tert.-Butyl | Öl | 2961, 2906, 2868, 2794, 1491, 1363, 1086, 1016, 830, 805 |
| 56 | 4-tert.-Butoxy-benzyl | tert.-Butyl | | |
| 57 | 1,4-Dioxa-spiro[4,5]decan-8-yl 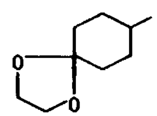 | tert.-Butyl | Harz | 2954, 2874, 2783, 1382, 1367, 1155, 1144, 1105, 1037, 923 |
| 58 | 3,3-Dimethyl-butyl | 2-Methyl-but-2-yl | | |
| 59 | 4,4-Dimethyl-pentyl | 2-Methyl-but-2-yl | | |
| 60 | 2,4,4-Trimethyl-pentyl | 2-Methyl-but-2-yl | | |
| 61 | 6-Methyl-hept-2-yl | 2-Methyl-but-2-yl | | |
| 62 | 6,10-Dimethyl-undec-2-yl | 2-Methyl-but-2-yl | | |

| Verb.-Nr. | $R^1$ | $R^2$ | Sdp.($^0$C/mbar) | IR-Absorption ($cm^{-1}$) [Film] |
|---|---|---|---|---|
| 63 | 3-Methyl-cyclohexyl | 2-Methyl-but-2-yl | | |
| 64 | 3,3-Dimethyl-cyclohexyl | 2-Methyl-but-2-yl | | |
| 65 | 3,5,5-Trimethyl-hexyl | 2-Methyl-but-2-yl | | |
| 66 | 4-Isopropyl-cyclohexyl | 2-Methyl-but-2-yl | | |
| 67 | 4-tert.-Butyl-cyclohexyl (Isomerengemisch) | 2-Methyl-but-2-yl | | |
| 68 | trans-4-tert.-Butyl-cyclohexyl | 2-Methyl-but-2-yl | | |
| 69 | 4-Trimethylsilylcyclohexyl | 2-Methyl-but-2-yl | | |
| 70 | 4-Hydroxy-cyclohexyl | 2-Methyl-but-2-yl | | |
| 71 | 3,3,5-Trimethyl-cyclohexyl | 2-Methyl-but-2-yl | | |
| 72 | 2-Decalyl | 2-Methyl-but-2-yl | | |
| 73 | 6-Hydroxy-2-decalyl | 2-Methyl-but-2-yl | | |
| 74 | 5,5,9-Trimethyl-2-decalyl | 2-Methyl-but-2-yl | | |
| 75 | 6-Hydroxy-5,9-dimethyl-2-decalyl | 2-Methyl-but-2-yl | | |
| 76 | 6-Hydroxy-5,5,9-Trimethyl-2-decalyl | 2-Methyl-but-2-yl | | |
| 77 | 4-Isopropyl-cyclohexyl | 2,4,4-Trimethyl-pent-2-yl | | |
| 78 | 3,3-dimethyl-cyclohexyl | 2,4,4-Trimethyl-pent-2-yl | | |
| 79 | 3,3-Dimethyl-butyl | 2,4,4-Trimethyl-pent-2-yl | | |
| 80 | 4-tert.-Butyl-cyclohexyl | 2,4,4-Trimethyl-pent-2-yl | | |
| 81 | 4-Trimethylsilyl-cyclohexyl | 2,4,4-Trimethyl-pent-2-yl | | |
| 82 | 2-Decalyl | 2,4,4-Trimethyl-pent-2-yl | | |
| 83 | 6-Hydroxy-2-decalyl | 2,4,4-Trimethyl-pent-2-yl | | |
| 84 | 5,5,9-Trimethyl-2-decalyl | 2,4,4-Trimethyl-pent-2-yl | | |
| 85 | 6-Hydroxy-5,9-dimethyl-decalyl | 2,4,4-Trimethyl-pent-2-yl | | |
| 86 | 3,3-Dimethylbutyl | tert.-Butoxy | | |
| 87 | 4,4-Dimethylpentyl | tert.-Butoxy | | |
| 88 | 2,4,4-Trimethyl-pentyl | tert.-Butoxy | | |
| 89 | 6-Methyl-hept-2-yl | tert.-Butoxy | | |
| 90 | 3,5,5-Trimethyl-hexyl | tert.-Butoxy | | |

| Verb.-Nr. | $R^1$ | $R^2$ | Sdp. (°C/mbar) | IR-Absorption ($cm^{-1}$) [Film] |
|---|---|---|---|---|
| 91 | 3-Methyl-cyclohexyl | tert.-Butoxy | | |
| 92 | 3,3-Dimethyl-cyclohexyl | tert.-Butoxy | | |
| 93 | 3,5,5-Trimethyl-cyclohexyl | tert.-Butoxy | | |
| 94 | 4-Isopropyl-cyclohexyl | tert.-Butoxy | | |
| 95 | 4-tert.-Butyl-cyclohexyl | tert.-Butoxy | | |
| 96 | 4-Trimethylsilyl-cyclohexyl | tert.-Butoxy | | |
| 97 | 2-Decalyl | tert.-Butoxy | | |
| 98 | 6-Hydroxy-2-decalyl | tert.-Butoxy | | |
| 99 | 5,5,9-Trimethyl-decalyl | tert.-Butoxy | | |
| 100 | 6-Hydroxy-5,9-trimethyl-decalyl | tert.-Butoxy | | |
| 101 | 4-Hydroxy-3,3,5-trimethyl-cyclohexyl | tert.-Butoxy | | |
| 102 | 4-tert.-Butyl-cyclohexyl | Trimethylsilyl | | |
| 103 | 3,3-Dimethylcyclohexyl | Trimethylsilyl | | |
| 104 | 2-Decalyl | Trimethylsilyl | | |
| 105 | 6-Hydroxy-2-decalyl | Trimethylsilyl | | |
| 106 | 3,3,5-Trimethyl-cyclohexyl | Trimethylsilyl | | |
| 107 | 5,5,9-Trimethyl-2-decalyl | Trimethylsilyl | | |

Tabelle 2

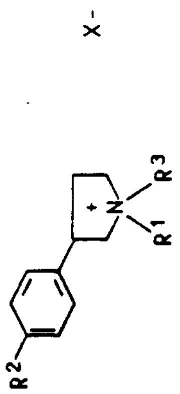

n = 1

| Verb.-Nr. | $R^1$ | $R^2$ | $R^3$ | $X^-$ |
|---|---|---|---|---|
| 108 | 4-tert.-Butyl-cyclohexyl | tert.-Butyl | Methyl | Jodid |
| 109 | 4-tert.-Butyl-cyclohexyl | tert.-Butyl | Methyl | Chlorid |
| 110 | 4-tert.-Butyl-cyclohexyl | tert.-Butyl | Benzyl | Chlorid |
| 111 | 2-Decalyl | tert.-Butyl | Methyl | Jodid |
| 112 | 6-Hydroxy-2-decalyl | tert.-Butyl | Methyl | Jodid |
| 113 | 3,3-Dimethyl-cyclohexyl | tert.-Butyl | Methyl | Jodid |
| 114 | 5,5,9-Trimethyl-2-decalyl | tert.-Butyl | Methyl | Jodid |
| 115 | 2,2-Dimethyl-propyl | tert.-Butyl | Methyl | Jodid |
| 116 | 4,4-Dimethyl-pentyl | tert.-Butyl | Methyl | Jodid |
| 117 | 2,4,4-Trimethyl-pentyl | tert.-Butyl | Methyl | Jodid |
| 118 | 4-Isopropyl-cyclohexyl | tert.-Butyl | Methyl | Jodid |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,

Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen.
Plasmopara viticola an Reben,
Alternaria-Arten an Obst und Gemüse.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Einige der neuen Verbindungen haben sehr gute Wirksamkeit gegen humanpathogene Pilze, wie Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 16a mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 20 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 16a werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 20 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 16a werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle

vermahlen. Durch feines Verteilen der Mischung in Wasser erhält man eine Spritzbrühe.

VI. 3 Gew.-Teile der Verbindung Nr. 20 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 16a werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 20 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 16a werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,

4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3--chlor-2-aminopyridin,
1-((bis-(4-Fluorphenyl)-methylsilyl)-methyl)-1H-1,2,4-triazol.

Anwendungsbeispiele

Als Vergleichswirkstoff wurde N(2-Methyl-3-(p-tert.-butyl-phenyl)propyl-1)-pyrrolidin (A) - bekannt aus DE 2 727 482 - benutzt.

Anwendungsbeispiel 1

Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 16a und 20 bei der Anwendung als 0,05 %ige Spritzbrühe eine bessere fungizide Wirkung zeigen (97 %) als der bekannte Vergleichswirkstoff A (50 %).

Anwendungsbeispiel 2

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Außmaßes des Pilzausbruches auf den Blattunterseiten.

Das Ergebnis zeigt, daß die Wirkstoffe 16a und 20 bei der Anwendung als 0,05 %ige Spritzbrühe eine bessere fungizide Wirkung zeigen (97 %) als der bekannte Vergleichswirkstoff A (60 %).

## Patentansprüche

**1.** N-Substituierte 3-Arylpyrrolidin-Derivate der Formel I

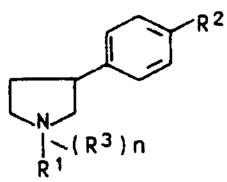

$(X^-)_n$ (I)

in der die Substituenten $R^1$ und $R^2$ die folgende Bedeutung haben:

$R^1$ bedeutet 2,2-Dimethyl-propyl, 3,3-Dimethyl-butyl, 4,4-Dimethyl-pentyl, 2,4,4-Trimethyl-pentyl, 6-Methyl-hept-2-yl, 3,5,5-Trimethyl-hexyl, 6,10-Dimethyl-undec-2-yl, 3-Methyl-cyclohexyl, 3,3-Dimethyl-cyclohexyl, 3,3,5-Trimethyl-cyclohexyl, 3,3,5,5-Tetramethyl-cyclohexyl, 4-Methyl-cyclohexyl, 4-Ethyl-cyclohexyl, 4-Propylcyclohexyl, 4-Isopropylcyclohexyl, 4-tert.-Butyl-cyclohexyl, trans-4-tert.-Butyl-cyclohexyl, 4(2-Methyl-but-2-yl)cyclohexyl, 4(2,4,4-Trimethyl-pent-2-yl)-cyclohexyl, Cyclododecanyl, durch C3-$C_9$-Trialkylsilyl-substituiertes $C_4$-$C_{12}$-Cycloalkyl, 4-Hydroxy-cyclohexyl, 4-Hydroxy-3-methyl-cyclohexyl, 4-Hydroxy-3,5-dimethyl-cyclohexyl, 4-Hydroxy-3,3-dimethyl-cyclohexyl,4-Hydroxy-3,3,5-trimethyl-cyclohexyl, gegebenenfalls substituiertes $C_5$-$C_{12}$-Cycloalkenyl mit den Substituenten Hydroxy, $C_1$-$C_9$-Alkyl, $C_1$-$C_5$-Alkoxy, $C_3$-$C_9$-Trialkylsilyl,

$R^1$ bedeutet ferner $C_9$-$C_{11}$-Bicycloalkyl, welches gegebenenfalls substituiert ist durch Hydroxy, $C_1$-$C_6$-Alkyl, $C_1$-$C_5$-Alkoxy, $C_3$-$C_9$-Trialkylsilyl, Acetoxy, Benzoyloxy, Benzyloxy,

$R^1$ bedeutet ferner 4-tert.-Butyl-benzyl, 4-Chlor-benzyl, 4-tert.-Butoxy-benzyl, 1,4-Dioxa-spiro[4,5]decan-8-yl,

5 bis 7-gliedriges Heterocycloalkyl mit 1 oder 2 Heteroatomen aus der Gruppe Sauerstoff und/oder Schwefel,

5 bis 7-gliedriges Heterocycloalkylmethyl mit 1 oder 2 Heteroatomen aus der Gruppe Sauerstoff

und/oder Schwefel, $C_1$-$C_8$-Alkyl-substituiertes 5 bis 7-gliedriges Heterocycloalkyl oder Heterocycloalkylmethyl mit 1 oder 2 Heteroatmen aus der Gruppe Sauerstoff und/oder Schwefel,

$R^2$ bedeutet $C_3$-$C_{10}$-Alkyl, $C_3$-$C_8$-Alkoxy, $C_3$-$C_9$-Trialkylsilyl,

$R^3$ bedeutet $C_1$-$C_5$-Alkvl, $C_3$-$C_6$-Alkenyl, $C_3$-$C_6$-Alkinyl, $C_7$-$C_{10}$-Arylalkyl, X- bedeutet ein für Pflanzen verträgliches Anion, n den Wert 0 oder 1 hat und deren für Pflanzen verträgliche Salze.

**2.** Verfahren zur Herstellung von Verbindungen der Formel I, dadurch gekennzeichnet, daß man

a) die Verbindung II

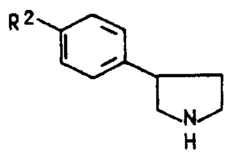

(II)

mit einer Carbonylverbindung III

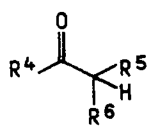

(III) ,

wobei die Reste $R^4$ bis $R^6$ so definiert sind, daß der Rest IV in seiner Gesamtheit dem Rest $R^1$ entspricht

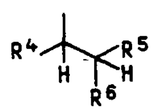

(IV),

direkt unter reduzierenden Bedingungen umsetzt oder zu einem Enamin umsetzt und dieses katalytisch hydriert oder mit einem Reduktionsmittel reduziert, oder

b) ein Phenylpyrrolidin-Derivat der Formel VI

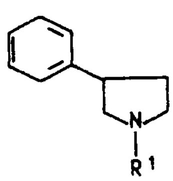

(VI)

säurekatalysiert mit einem Alken umsetzt, das in seiner Struktur dem Rest $R^2$ ist $C_3$-$C_{10}$-Alkyl entspricht und gegebenenfalls das gemäß a) oder b) erhaltene Pyrrolidinderivat mit einer Verbindung der Formel

$R^3$-X, in der der Substituent X die oben angegebene Bedeutung hat, umsetzt und anschließend gegebenenfalls das vorliegende Anion $X^-$ gegen ein anderes Anion $X^-$ austauscht.

**3.** Verfahren zur Herstellung der Verbindung

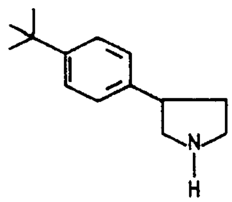

dadurch gekennzeichnet, daß man 3-Phenylpyrrolidin

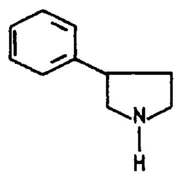

Säurekatalysiert mit Isobutylen umsetzt.

**4.** Fungizid, enthaltend eine fungizid wirksame Menge einer Verbindung der Formel I

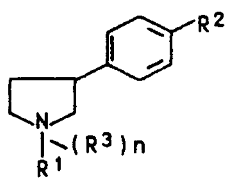

(I) $(X^-)_n$

in der die Substituenten $R^1$ und $R^2$ die folgende Bedeutung haben:

$R^1$ bedeutet 2,2-Dimethyl-propyl, 3,3-Dimethyl-butyl, 4,4-Dimethyl-pentyl, 2,4,4-Trimethyl-pentyl, 6-Methyl-hept-2-yl, 3,5,5-Trimethyl-hexyl, 6,10-Dimethyl-undec-2-yl, 3-Methyl-cyclohexyl, 3,3-Dimethyl-cyclohexyl, 3,3,5-Trimethyl-cyclohexyl, 3,3,5,5-Tetramethyl-cyclohexyl, 4-Methyl-cyclohexyl, 4-Ethyl-cyclohexyl, 4-Propylcyclohexyl, 4-Isopropylcyclohexyl, 4-tert.-Butyl-cyclohexyl, trans-4-tert.-Butyl-cyclohexyl, 4(2-Methyl-but-2-yl)cyclohexyl, 4(2,4,4-Trimethyl-pent-2-yl)cyclohexyl, Cyclododecanyl, durch C3-$C_9$-Trialkylsilyl-substituiertes $C_4$-$C_{12}$-Cycloalkyl, 4-Hydroxy-cyclohexyl, 4-Hydroxy-3-methyl-cyclohexyl, 4-Hydroxy-3,5-dimethyl-cyclohexyl, 4-Hydroxy-3,3-dimethyl-cyclohexyl, 4-Hydroxy-3,3,5-trimethyl-cyclohexyl, gegebenenfalls substituiertes $C_5$-$C_{12}$-Cycloalkenyl mit den Substituenten Hydroxy, $C_1$-$C_9$-Alkyl, $C_1$-$C_5$-Alkoxy, $C_3$-$C_9$-Trialkylsilyl,

$R^1$ bedeutet ferner $C_9$-$C_{11}$-Bicycloalkyl, welches gegebenenfalls substituiert ist durch Hydroxy, $C_1$-$C_6$-Alkyl, $C_1$-$C_5$-Alkoxy, $C_3$-$C_9$-Trialkylsilyl, Acetoxy, Benzoyloxy, Benzyloxy,

$R^1$ bedeutet ferner 4-tert.-Butyl-benzyl, 4-Chlor-benzyl, 4-tert.-Butoxy-benzyl, 1,4-Dioxa-spiro[4,5]decan-8-yl,

5 bis 7-gliedriges Heterocycloalkyl mit 1 oder 2 Heteroatomen aus der Gruppe Sauerstoff und/oder Schwefel,

5 bis 7-gliedriges Heterocycloalkylmethyl mit 1 oder 2 Heteroatomen aus der Grupe Sauerstoff und/oder Schwefel, $C_1$-$C_8$-Alkyl-substituiertes 5 bis 7-gliedriges Heterocycloalkyl oder Heterocycloalkylmethyl mit 1 oder 2 Heteroatmen aus der Gruppe Sauerstoff und/oder Schwefel,

$R^2$ bedeutet $C_3$-$C_{10}$-Alkyl, $C_3$-$C_8$-Alkoxy, $C_3$-$C_9$-Trialkylsilyl,

$R^3$ bedeutet $C_1$-$C_5$-Alkyl, $C_3$-$C_6$-Alkenyl, $C_3$-$C_6$-Alkinyl.

$C_7$-$C_{10}$-Arylalkyl, X- bedeutet ein für Planzen verträgliches Anion, n den Wert 0 oder 1 hat oder deren für Pflanzen verträgliche Salze und einen inerten Trägerstoff.

5. Verwendung einer Verbindungen der Formel I als Fungizid.

6. Verfahren zur Bekämpfung von Pilzen, dadurch gekennzeichnet, daß man die Pilze oder die vom Pilzbefall bedrohten Materialien, Planzen, Saatgüter oder den Boden mit einer fungizid wirksamen Menge einer Verbindung der Formel I

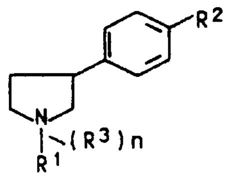

(I) $(X^-)_n$

in der die Substituenten $R^1$ und $R^2$ die folgende Bedeutung haben:

$R^1$ bedeutet 2,2-Dimethyl-propyl, 3,3-Dimethyl-butyl, 4,4-Dimethyl-pentyl, 2,4,4-Trimethyl-pentyl, 6-Methyl-hept-2-yl, 3,5,5-Trimethyl-hexyl, 6,10-Dimethyl-undec-2-yl, 3-Methyl-cyclohexyl, 3,3-Dimethyl-cyclohexyl, 3,3,5-Trimethyl-cyclohexyl.3,3,5,5-Tetramethyl-cyclohexyl, 4-Methyl-cyclohexyl, 4-Ethyl-cyclohexyl, 4-Propylcyclohexyl, 4-Isopropylcyclohexyl, 4-tert.-Butyl-cyclohexyl, trans-4-tert.-Butyl-cyclohexyl, 4(2-Methyl-but-2-yl)cyclohexyl, 4(2,4,4-Trimethyl-pent-2-yl)-cyclohexyl, Cyclododecanyl, durch C3-$C_9$-Trialkylsilyl-substituiertes $C_4$-$C_{12}$-Cycloalkyl, 4-Hydroxy-cyclohexyl, 4-Hydroxy-3-methyl-cyclohexyl, 4-Hydroxy-3,5-dimethyl-cyclohexyl, 4-Hydroxy-3,3-dimethyl-cyclohexyl, 4-Hydroxy-3,3,5-trimethyl-cyclohexyl, gegebenenfalls substituiertes $C_5$-$C_{12}$-Cycloalkenyl mit den Substituenten Hydroxy, $C_1$-$C_9$-Alkyl, $C_1$-$C_5$-Alkoxy, $C_3$-$C_9$-Trialkylsilyl,

$R^1$ bedeutet ferner $C_9$-$C_{11}$-Bicycloalkyl, welches gegebenenfalls substituiert ist durch Hydroxy, $C_1$-$C_6$-Alkyl, $C_1$-$C_5$-Alkoxy, $C_3$-$C_9$-Trialkylsilyl, Acetoxy, Benzoyloxy, Benzyloxy,

$R^1$ bedeutet ferner 4-tert.-Butyl-benzyl, 4-Chlor-benzyl, 4-tert.-Butoxy-benzyl, 1,4-Dioxa-spiro[4,5]decan-8-yl,

5 bis 7-gliedriges Heterocycloalkyl mit 1 oder 2 Heteroatomen aus der Gruppe Sauerstoff und/oder Schwefel,

5 bis 7-gliedriges Heterocycloalkylmethyl mit 1 oder 2 Heteroatomen aus der Grupe Sauerstoff und/oder Schwefel, $C_1$-$C_8$-Alkyl-substituiertes 5 bis 7-gliedriges Heterocycloalkyl oder Heterocycloalkylmethyl mit 1 oder 2 Heteroatmen aus der Gruppe Sauerstoff und/oder Schwefel,

$R^2$ bedeutet $C_3$-$C_{10}$-Alkyl, $C_3$-$C_8$-Alkoxy, $C_3$-$C_9$-Trialkylsilyl,

$R^3$ bedeutet $C_1$-$C_5$-Alkyl, $C_3$-$C_6$-Alkenyl, $C_3$-$C_6$-Alkinyl, $C_7$-$C_{10}$-Arylalkyl, $X^-$ bedeutet ein für Pflanzen vertägliches Anion, n den Wert 0 oder 1 hat oder deren für Pflanzen verträglichen Salzen behandelt.

7. Verbindungen gemäß Anspruch 1, nämlich N-(3,3-Dimethyl-cyclohexyl)-3-(4-tert.-butyl-phenyl)-pyrrolidin.

8. Verbindungen gemäß Anspruch 1, nämlich N-(2-Decalyl)-3-(4-tert-butyl-phenyl)) pyrrolidin.

9. Verbindungen gemäß Anspruch 1, nämlich N-(3,3-Dimethyl-butyl)-3-(4-tert.-butyl-phenyl)-pyrrolidin.

10. Verbindungen gemäß Anspruch 1, nämlich N-(4,4-Dimethyl-pentyl)-3-(4-tert.-butyl-phenyl)-pyrrolidin.

## Claims

1. An N-substituted 3-arylpyrrolidine derivative of the formula I

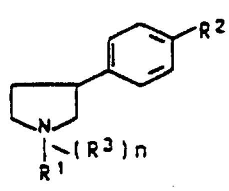

$(X^-)_n$ (I)

where $R^1$ is 2,2-dimethylpropyl, 3,3-dimethylbutyl, 4,4-dimethylpentyl, 2,4,4-trimethylpentyl, 6-methylhept-2-yl, 3,5,5-trimethylhexyl, 6,10-dimethylundec-2-yl, 3-methylcyclohexyl, 3,3-dimethylcyclohexyl, 3,3,5-trimethylcyclohexyl, 3,3,5,5-tetramethylcyclohexyl, 4-methylcyclohexyl, 4-ethylcyclohexyl, 4-propylcyclohexyl, 4-isopropylcyclohexyl, 4-tert-butylcyclohexyl, trans-4-tert-butylcyclohexyl, 4-(2-methylbut-2-yl)-cyclohexyl, 4-(2,4,4-trimethylpent-2-yl)-cyclohexyl, cyclododecanyl, $C_3$-$C_9$-trialkylsilyl-substituted $C_4$-$C_{12}$-cycloalkyl, 4-hydroxycyclohexyl, 4-hydroxy-3-methylcyclohexyl, 4-hydroxy-3,5-dimethylcyclohexyl, 4-hydroxy-3,3-dimethylcyclohexyl, 4-hydroxy-3,3,5-trimethylcyclohexyl, unsubstituted or hydroxy-, $C_1$-$C_9$-alkyl-, $C_1$-$C_5$-alkoxy- or $C_3$-$C_9$-trialkylsilyl-substituted $C_5$-$C_{12}$-cycloalkenyl,
$R^1$ is further $C_9$-$C_{11}$-bicycloalkyl which is unsubstituted or substituted by hydroxy, $C_1$-$C_6$-alkyl, $C_1$-$C_5$-alkoxy, $C_3$-$C_9$-trialkylsilyl, acetoxy, benzoyloxy or benzyloxy,
$R^1$ is further 4-tert-butylbenzyl, 4-chlorobenzyl, 4-tert-butoxybenzyl, 1,4-dioxaspiro[4,5]decan-8-yl,
5- to 7-membered heterocycloalkyl having 1 or 2 heteroatoms selected from the group consisting of oxygen and/or sulfur,
5- to 7-membered heterocycloalkylmethyl having 1 or 2 heteroatoms selected from the group consisting of oxygen and/or sulfur, $C_1$-$C_8$-alkyl-substituted 5- to 7-membered heterocycloalkyl or heterocycloalkylmethyl having 1 or 2 heteroatoms selected from the group consisting of oxygen and/or sulfur,
$R^2$ is $C_3$-$C_{10}$-alkyl, $C_3$-$C_8$-alkoxy or $C_3$-$C_9$-trialkylsilyl,
$R^3$ is $C_1$-$C_5$-alkyl, $C_3$-$C_6$-alkenyl, $C_3$-$C_6$-alkynyl or $C_7$-$C_{10}$-arylalkyl,
X- is a plant-tolerated anion,
n is 0 or 1,
or a plant-tolerated salt thereof.

**2.** A process for the preparation of a compound of the formula I, wherein
a) a compound II

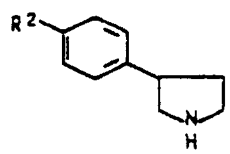

(II)

is reacted with a carbonyl compound III

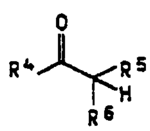

(III) ,

where $R^4$ to $R^6$ are defined so that the radical IV corresponds in its totality to $R^1$,

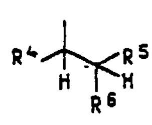

(IV) ,

directly under reducing conditions or is converted into an enamine, and the latter is catalytically hydrogenated or is reduced with a reducing agent, or

b) a phenylpyrrolidine derivative of the formula VI

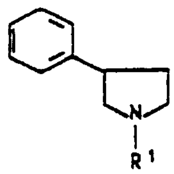

(VI)

is subjected to an acid-catalyzed reaction with an alkene whose structure corresponds to the radical $R^2$ is $C_3$-$C_{10}$-alkyl and, if required, the pyrrolidine derivative obtained according to a) or b) is reacted with a compound of the formula $R^3$-X, where X has the abovementioned meaning, and then, if required, the anion $X^-$ present is exchanged for another anion $X^-$.

**3.** A process for the preparation of the compound

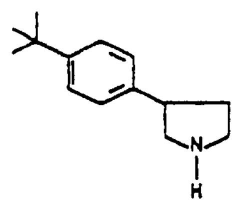

wherein 3-phenylpyrrolidine

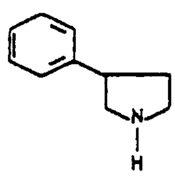

is subjected to an acid-catalyzed reaction with isobutylene.

**4.** A fungicide containing a fungicidally effective amount of a compound of the formula I

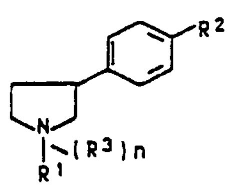

$(X^-)_n$ (I)

where $R^1$ is 2,2-dimethylpropyl, 3,3-dimethylbutyl, 4,4-dimethylpentyl, 2,4,4-trimethylpentyl, 6-methylhept-2-yl, 3,5,5-trimethylhexyl, 6,10-dimethylundec-2-yl, 3-methylcyclohexyl, 3,3-dimethylcyclohexyl, 3,3,5-trimethylcyclohexyl, 3,3,5,5-tetramethylcyclohexyl, 4-methylcyclohexyl, 4-ethylcyclohexyl, 4-propylcyclohexyl, 4-isopropylcyclohexyl, 4-tert-butylcyclohexyl, trans-4-tert-butylcyclohexyl, 4-(2-methylbut-2-yl)-cyclohexyl, 4-(2,4,4-trimethylpent-2-yl)-cyclohexyl, cyclododecanyl, $C_3$-$C_9$-trialkylsilyl-substituted $C_4$-$C_{12}$-cycloalkyl, 4-hydroxycyclohexyl, 4-hydroxy-3-methylcyclohexyl, 4-hydroxy-3,5-dimethylcyclohexyl, 4-hydroxy-3,3-dimethylcyclohexyl, 4-hydroxy-3,3,5-trimethylcyclohexyl, unsubstituted or hydroxy-, $C_1$-$C_9$-alkyl-, $C_1$-$C_5$-alkoxy- or $C_3$-$C_9$-trialkylsilyl-substituted $C_5$-$C_{12}$-cycloalkenyl,
$R^1$ is further $C_9$-$C_{11}$-bicycloalkyl which is unsubstituted or substituted by hydroxy, $C_1$-$C_6$-alkyl, $C_1$-$C_5$-alkoxy, $C_3$-$C_9$-trialkylsilyl, acetoxy, benzoyloxy or benzyloxy,
$R^1$ is further 4-tert-butylbenzyl, 4-chlorobenzyl, 4-tert-butoxybenzyl, 1,4-dioxaspiro[4,5]decan-8-yl,
5- to 7-membered heterocycloalkyl having 1 or 2 heteroatoms selected from the group consisting of

oxygen and/or sulfur,
5- to 7-membered heterocycloalkylmethyl having 1 or 2 heteroatoms selected from the group consisting of oxygen and/or sulfur, $C_1$-$C_8$-alkyl-substituted 5- to 7-membered heterocycloalkyl or heterocycloalkylmethyl having 1 or 2 heteroatoms selected from the group consisting of oxygen and/or sulfur,
$R^2$ is $C_3$-$C_{10}$-alkyl, $C_3$-$C_8$-alkoxy or $C_3$-$C_9$-trialkylsilyl,
$R^3$ is $C_1$-$C_5$-alkyl, $C_3$-$C_6$-alkenyl, $C_3$-$C_6$-alkynyl or $C_7$-$C_{10}$-arylalkyl,
X- is a plant-tolerated anion,
n is 0 or 1,
or a plant-tolerated salt thereof, and an inert carrier.

**5.** Use of a compound of the formula I as a fungicide.

**6.** A method for controlling fungi, wherein the fungi or the materials, plants, seed or soil threatened by fungal attack are or is treated with a fungicidally effective amount of a compound of the formula I

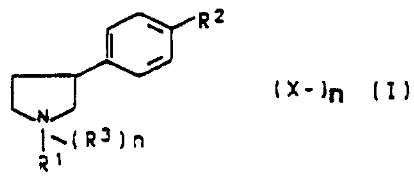

where $R^1$ is 2,2-dimethylpropyl, 3,3-dimethylbutyl, 4,4-dimethylpentyl, 2,4,4-trimethylpentyl, 6-methylhept-2-yl, 3,5,5-trimethylhexyl, 6,10-dimethylundec-2-yl, 3-methylcyclohexyl, 3,3-dimethylcyclohexyl, 3,3,5-trimethylcyclohexyl, 3,3,5,5-tetramethylcyclohexyl, 4-methylcyclohexyl, 4-ethylcyclohexyl, 4-propylcyclohexyl, 4-isopropylcyclohexyl, 4-tert-butylcyclohexyl, trans-4-tert-butylcyclohexyl, 4-(2-methylbut-2-yl)-cyclohexyl, 4-(2,4,4-trimethylpent-2-yl)-cyclohexyl, cyclododecanyl, $C_3$-$C_9$-trialkylsilyl-substituted $C_4$-$C_{12}$-cycloalkyl, 4-hydroxycyclohexyl, 4-hydroxy-3-methylcyclohexyl, 4-hydroxy-3,5-dimethylcyclohexyl, 4-hydroxy-3,3-dimethylcyclohexyl, 4-hydroxy-3,3,5-trimethylcyclohexyl, unsubstituted or hydroxy-, $C_1$-$C_9$-alkyl-, $C_1$-$C_5$-alkoxy- or $C_3$-$C_9$-trialkylsilyl-substituted $C_5$-$C_{12}$-cycloalkenyl,
$R^1$ is further $C_9$-$C_{11}$-bicycloalkyl which is unsubstituted or substituted by hydroxy, $C_1$-$C_6$-alkyl, $C_1$-$C_5$-alkoxy, $C_3$-$C_9$-trialkylsilyl, acetoxy, benzoyloxy or benzyloxy,
$R^1$ is further 4-tert-butylbenzyl, 4-chlorobenzyl, 4-tert-butoxybenzyl, 1,4-dioxaspiro[4,5]decan-8-yl,
5- to 7-membered heterocycloalkyl having 1 or 2 heteroatoms selected from the group consisting of oxygen and/or sulfur,
5- to 7-membered heterocycloalkylmethyl having 1 or 2 heteroatoms selected from the group consisting of oxygen and/or sulfur, $C_1$-$C_8$-alkyl-substituted 5- to 7-membered heterocycloalkyl or heterocycloalkylmethyl having 1 or 2 heteroatoms selected from the group consisting of oxygen and/or sulfur,
$R^2$ is $C_3$-$C_{10}$-alkyl, $C_3$-$C_8$-alkoxy or $C_3$-$C_9$-trialkylsilyl,
$R^3$ is $C_1$-$C_5$-alkyl, $C_3$-$C_6$-alkenyl, $C_3$-$C_6$-alkynyl or $C_7$-$C_{10}$-arylalkyl,
X- is a plant-tolerated anion,
n is 0 or 1,
or a plant-tolerated salt thereof.

**7.** A compound as claimed in claim 1, namely N-(3,3-dimethylcyclohexyl)-3-(4-tert-butylphenyl)-pyrrolidine.

**8.** A compound as claimed in claim 1, namely N-(2-decalyl)-3-(4-tert-butylphenyl)-pyrrolidine.

**9.** A compound as claimed in claim 1, namely N-(3,3-dimethylbutyl)-3-(4-tert-butylphenyl)-pyrrolidine.

**10.** A compound as claimed in claim 1, namely N-(4,4-dimethylpentyl)-3-(4-tert-butylphenyl)-pyrrolidine.

**Revendications**

1. Dérivés de 3-aryl-pyrrolidine substitués sur N de formule I

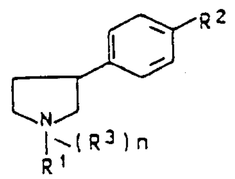

$(X^-)_n$ (I)

dans laquelle les substituants $R^1$ et $R^2$ ont les significations suivantes :
$R^1$ représente 2,2-diméthyl-propyle, 3,3-diméthyl-butyle, 4,4-diméthyl-pentyle, 2,4,4-trimétnyl-pentyle, 6-méthyl-hept-2-yle, 3,5,5-trimétnyl-hexyle, 6,10-diméthyl-undec-2-yle, 3-méthyl-cyclohexyle, 3,3-diméthyl-cyclohexyle, 3,3,5-triméthyl-cyclohexyle, 3,3,5,5-tétraméthyl-cyclohexyle, 4-méthyl-cyclohexyle, 4-éthyl-cyclohexyle, 4-propylcyclohexyle, 4-isopropylcyclohexyle, 4-tert.-butyl-cyclohexyle, trans-4-tert.-butyl-cyclohexyle, 4(2-méthyl-but-2-yl)-cyclohexyle, 4(2,4,4-trimétyl-pent-2-yl)-cyclohexyle, cyclo-dodécanyle, C4-C12-cyclo-alkyle substitués par C3-C9-trialkylsilyle, 4-hydroxy-cyclohexyle, 4-hydroxy-3-méthyl-cyclohexyle, 4-hydroxy-3,5-diméthyl-cyclohexyle, 4-hydroxy-3,3-diméthyl-cyclonexyle, 4-hydroxy-3,3,5-triméthyl-cyclohexyle, cycloalcényle en C5-C12, éventuellement substitué par les substituants hydroxy, alkyle en C1-C9, alcoxy en C1-C5, trialkylsilyle en C3-C9,
$R^1$ représente en outre bicycloalkyle en C9-C11, éventuellement substitué par hydroxy, alkyle en C1-C6, alcoxy en C1-C5, trialkylsilyle en C3-C9, acétoxy, benzoyloxy, benzyloxy,
$R^1$ représente en outre 4-ter-butyl-benzyle, 4-chloro-benzyle, 4-ter-butoxy-benzyle, 1,4-dioxa-spiro [4,5]-décane-8-yle,
hétérocycloalkyle à 5 à 7 chaînons, portant 1 ou 2 hétéroatomes choisis dans le groupe oxygène et/ou soufre,
hétéroxycloalkylméthyle à 5 à 7 chaînons, portant 1 ou 2 hétéro-atomes choisis dans le groupe oxygène et/ou soufre, hétérocycloalkyle ou hétérocycloalkylméthyle à 5 ou 7 chaînons, substitués par alkyle en C1-C8 et portant 1 ou 2 hétéroatomes choisis dans le groupe oxygène et/ou soufre.
$R^2$ représente alkyle en C3-C10, alcoxy en C3-C8, trialkylsilyle en C3-C9,
$R^3$ représente alkyle en C1-C5, alcényle en C3-C6,alcynyle en C3-C6, arylalkyle en C7-C10, X-représentant un anion toléré par les plantes, n ayant la valeur 0 ou 1,
ainsi que leurs sels acceptables pour les plantes.

2. Procédé de préparation de composés de formule I, caractérisé par le fait que l'on met à réagir directement, dans des conditions réductrices,
   a) le composé II

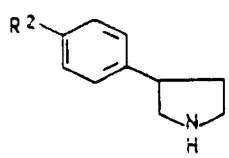

(II)

avec un composé carbonylé III

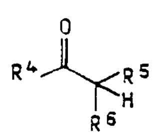

(III) ,

les restes $R^4$ à $R^6$ étant définis de manière telle que le reste IV correspond dans son ensemble, au reste $R^1$

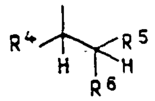

(IV),

ou on le transforme en énamine et on soumet celle-ci à un hydrogénation catalytique ou on la réduit avec un agent réducteur, ou

b) on fait réagir, avec catalyse par un acide, un dérivé de phénylpyrrolidine de formule VI

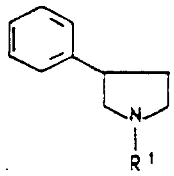

(VI)

avec un alcène dont la structure correspond au reste $R^2$, à savoir alkyle en C3-C10, et on fait éventuellement réagir le dérivé de pyrrolidine obtenu conformément à a) ou b), avec un composé de formule $R^3$-X, dans laquelle le substituant X a la signification donnée plus haut, ensuite on échange éventuellement l'anion $X^-$ présent contre un autre anion $X^-$.

**3.** Procédé de préparation du composé

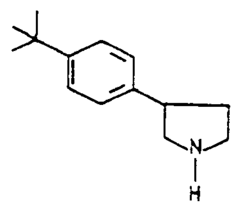

caractérisé par le fait que l'on fait réagir la 3-phénylpyrrolidine

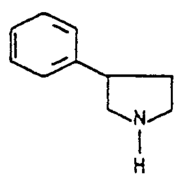

avec catalyse par un acide, avec de l'isobutylène.

**4.** Fongicide, contenant une quantité efficace au point de vue fongicide, d'un composé de formule I

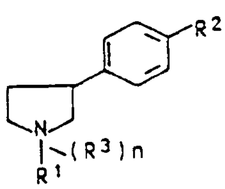

(I) $(X^-)_n$

dans laquelle les substituants $R^1$ et $R^2$ ont les significations suivantes :

$R^1$ représente 2,2-diméthyl-propyle, 3,3-diméthyl-butyle, 4,4-diméthyl-pentyle, 2,4,4-triméthylpentyle, 6-méthyl-hept-2-yle, 3,5,5-triméthyl-hexyle, 6,10-diméthyl-undec-2-yle, 3-méthyl-cyclohexyle, 3,3-diméthyl-cyclohexyle, 3,3,5-triméthyl-cyclohexyle, 3,3,5,5-tétraméthyl-cyclohexyle, 4-méthyl-cyclohexyle, 4-éthyl-cyclohexyle, 4-propylcyclohexyle, 4-isopropylcyclohexyle, 4-tert.-butyl-cyclohexyle, trans-4-tert.-butyl-cyclohexyle, 4(2-méthyl-but-2-yl)-cyclohexyle, 4(2,4,4-trimétyl-pent-2-yl)-cyclohexyle, cyclo-dodécanyle, C4-C12-cyclo-alkyle substitués par C3-C9-trialkylsilyle, 4-hydroxy-cyclohexyle, 4-hydroxy-3-méthyl-cyclohexyle, 4-hydroxy-3,5-diméthyl-cyclohexyle, 4-hydroxy-3,3-diméthyl-cyclohexyle, 4-hydroxy-3,3,5-triméthyl-cyclohexyle, cycloalcényle en C5-C12, éventuellement substitué par les substituants hydroxy, alkyle en C1-C9, alcoxy en C1-C5, trialkylsilyle en C3-C9,

$R^1$ représente en outre bicycloalkyle en C9-C11, éventuellement substitué par hydroxy, alkyle en C1-C6, alcoxy en C1-C5, trialkylsilyle en C3-C9, acétoxy, benzoyloxy, benzyloxy,

$R^1$ représente en outre 4-ter-butyl-benzyle, 4-chloro-benzyle, 4-ter-butoxy-benzyle, 1,4-dioxa-spiro [4,5] décane-8-yle,

hétérocycloalkyle à 5 à 7 chaînons, portant 1 ou 2 hétéroatomes choisis dans le groupe oxygène et/ou soufre,

hétéroxycloalkylméthyle à 5 à 7 chaînons, portant 1 ou 2 hétéro-atomes choisis dans le groupe oxygéne et/ou soufre, hétérocycloalkyle ou hétérocycloalkylméthyle à 5 ou 7 chaînons, substitués par alkyle en C1-C8 et portant 1 ou 2 hétéroatomes choisis dans le groupe oxygène et/ou soufre.

$R^2$ représente alkyle en C3-C10, alcoxy en C3-C8, trialkylsilyle en C3-C9,

$R^3$ représente alkyle en C1-C5, alcényle en C3-C6,alcynyle en C3-C6, arylalkyle en C7-C10, X- représentant un anion toléré par les plantes, n ayant la valeur 0 ou 1,

ainsi que leurs sels acceptables pour les plantes.

5. Utilisation d'un composé de formule I comme fongicide.

6. Procédé de lutte contre les champignons, caractérisé par le fait que l'on traite les champignons ou les matériaux, plantes, semences ou le sol avec une quantité efficace au point de vue fongicide d'un composé de formule I

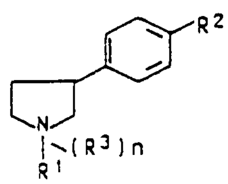

(I) $(X^-)_n$

dans laquelle les substituants $R^1$ et $R^2$ ont les significations suivantes :

$R^1$ représente 2,2-diméthyl-propyle, 3,3-diméthyl-butyle, 4,4-diméthyl-pentyle, 2,4,4-triméthyl-pentyle, 6-méthyl-hept-2-yle, 3,5,5-triméthyl-hexyle, 6,10-diméthyl-undec-2-yle, 3-méthyl-cyclohexyle, 3,3-diméthyl-cyclohexyle, 3,3,5-triméthyl-cyclohexyle, 3,3,5,5-tétraméthyl-cyclohexyle, 4-méthyl-cyclohexyle, 4-éthyl-cyclohexyle, 4-propylcyclohexyle, 4-isopropylcyclohexyle, 4-tert.-butyl-cyclohexyle, trans-4-tert.-butyl-cyclohexyle, 4(2-méthyl-but-2-yl)-cyclohexyle, 4(2,4,4-trimétyl-pent-2-yl)-cyclohexyle, cyclo-dodécanyle, C4-C12-cyclo-alkyle substitués par C3-C9-trialkylsilyle, 4-hydroxy-cyclohexyle, 4-hydroxy-3-méthyl-cyclohexyle, 4-hydroxy-3,5-diméthyl-cyclohexyle, 4-hydroxy-3,3-diméthyl-cyclohexyle, 4-hydroxy-3,3,5-triméthyl-cyclohexyle, cycloalcényle en C5-C12, éventuellement substitué par les substituants hydroxy, alkyle en C1-C9, alcoxy en C1-C5, trialkylsilyle en C3-C9,

$R^1$ représente en outre bicycloalkyle en C9-C11, éventuellement substitué par hydroxy, alkyle en C1-C6, alcoxy en C1-C5, trialkylsilyle en C3-C9, acétoxy, benzoyloxy, benzyloxy,

$R^1$ représente en outre 4-ter-butyl-benzyle, 4-chloro-benzyle, 4-ter-butoxy-benzyle, 1,4-dioxa-spiro [4,5] décane-8-yle,

hétérocycloalkyle à 5 à 7 chaînons, portant 1 ou 2 hétéroatomes choisis dans le groupe oxygène et/ou soufre,

hétéroxycloalkylméthyle à 5 à 7 chaînons, portant 1 ou 2 hétéro-atomes choisis dans le groupe oxygène et/ou soufre, hétérocycloalkyle ou hétérocycloalkylméthyle à 5 ou 7 chaînons, substitués par alkyle en C1-C8 et partant 1 ou 2 hétéroatomes choisis dans le groupe oxygène et/ou soufre.

$R^2$ représente alkyle en C3-C10, alcoxy en C3-C8, trialkylsilyle en C3-C9,

$R^3$ représente alkyle en C1-C5, alcényle en C3-C6,alcynyle en C3-C6, arylalkyle en C7-C10, X- représentant un anion toléré par les plantes, n ayant la valeur 0 ou 1,

ainsi que leurs sels acceptables pour les plantes.

**7.** Composé selon la revendication 1, a savoir N-(3,3-diméthyl-cyclohexyl)-3-(4-ter-butyl-péhyl)-pyrrolidine.

**8.** Composé selon la revendication 1, à savoir N-(2-décalyl)-3-(4-ter-butyl-phényl)pyrrolidine.

**9.** Composé selon la revendication 1, àsavoir N-(3,3-diméthyl-butyl)-3-(4-ter-butyl-phényl)-pyrrolidine.

**10.** Composé selon la revendication 1, à savoir N-(4,4-diméthyl-pentyl)-3-(4-ter-butyl-phényl)-pyrrolidine.